# EUROPÄISCHE PATENTSCHRIFT

(11) **EP 2 467 190 B1**
(45) Veröffentlichungstag und Bekanntmachung des Hinweises auf die Patenterteilung: **05.03.2014**
(21) Anmeldenummer: 10732290.1
(22) Anmeldetag: 30.06.2010
(51) Int. Cl.: A61N 1/05, A61N 1/32

(54) **POLARISATIONSVORRICHTUNG UND IMPLANTATIONSSYSTEM**
POLARIZATION DEVICE AND IMPLANTATION SYSTEM
DISPOSITIF DE POLARISATION ET SYSTÈME D'IMPLANTATION

(30) Priorität: 30.06.2009 DE 102009031134
(43) Veröffentlichungstag der Anmeldung: 27.06.2012
(73) Patentinhaber: Geiges, Bjarne, 80538 München (DE)
(72) Erfinder: KRAUS, Werner, verstorben (DE)
(74) Vertreter: Schumacher & Willsau
(86) Internationale Anmeldenummer: PCT/EP2010/003954
(87) Internationale Veröffentlichungsnummer: WO 2011/000556

(56) Entgegenhaltungen:
- EP-A2- 0 108 383
- DE-A1- 2 311 817
- DE-A1- 3 942 735
- DE-A1- 4 230 181

## Beschreibung

Die Erfindung betrifft eine Vorrichtung zum Polarisieren von Implantaten sowie ein Implantationssystem mit einer solchen Vorrichtung.

Polarisationsvorrichtungen und Implantationssysteme sind auf dem Gebiet der Osteosynthese bekannt. Die Osteosynthese dient der belastungsstabilen Fixation der Fragmente eines gebrochenen oder kranken Knochens in seiner unverletzten, natürlichen Form durch implantierte Schrauben, Stützplatten, Drähte, Knochenmarknägel und dergleichen, die im Allgemeinen aus nicht rostenden Stahl- oder Titanlegierungen gefertigt sind. Diese Osteosynthesemittel ermöglichen die rasche Mobilisierung des Patienten bei gleichzeitiger Ruhigstellung des lädierten Knochens, die eine unerlässliche Voraussetzung für seine Heilung ist.

Problematisch an der starren Fixierung durch die vergleichsweise unelastischen, gewebeverdrängenden Stützimplantate ist jedoch die Behinderung der biologischen Erholung vor allem durch den Verlust von Blutgefäßen und Nerven. Außerdem leidet mit zunehmender Implantationsdauer die biomechanische Qualität der Stützstruktur durch den partiellen Entzug ihrer Funktion. Mit dem Verlust an biologischer Kontrolle aber wächst die Gefahr der Infektion durch resistente Bakterien (MRSA = Multiresistenter Staphylococcus Aureus). Es wurde gezeigt, dass diese die Oberfläche von Metallimplantaten in Form eines adhärenten Biofilmes besiedeln können und mit einer Schleimhülle aus Polysacchariden dem Angriff von Antibiotika widerstehen.

Diesen Problemen kann im Rahmen der orthopädischen Chirurgie durch die Elektro-Osteotherapie begegnet werden, beispielsweise unter Verwendung der eingangs genannten gattungsgemäßen Kontaktvorrichtung, wie beispielsweise in der US 6,778,861 B1 beschrieben. Bei der dort dargestellten magnetisch induzierten Elektro-Osteotherapie werden in Osteosythesemitteln elektrische Wechselpotentiale niedriger Frequenz dadurch induziert, dass ein betroffener Körperteil einem magnetischen Wechselfeld ausgesetzt wird. Seit langem wurde in zahlreichen klinischen Anwendungen der verfahrensgemäßen Technik bei chronisch therapieresistenten, meist infizierten Knochendefekten, Zysten und Tumormetastasen sowie in kliniknahen experimentellen Studien gezeigt, dass mit der Verwendung der Osteosynthese-Implantate als Quellen extrem niederfrequenter sinusförmiger elektrischer Wechselpotentiale in der dem Stützmetall anliegenden Knochenregion ein optimaler Heilungseffekt erzielt wird.

Die Technik der Übertragung funktioniert nach dem Transformatorprinzip: Die verletzte oder kranke Körperregion wird von einem extrem niederfrequenten sinusförmig verlaufenden Magnetfeld mit einer Frequenz von ca. 1 bis 100 Hz - vorzugsweise von 4 bis 20 Hz - und einer magnetischen Flussdichte von 0,5 bis 5 mT (5 bis 50 Gauß) durchflutet, das durch einen Funktionsstromgenerator in einer oder mehreren - primärenäußeren Stromspulen erzeugt wird, in die das mit den Osteosynthesemitteln versehene Körperteil eingebracht wird. Diese extrem niederfrequenten elektromagnetischen Felder durchdringen weitgehend verlustfrei das Gewebe, einschließlich eventuelle Kleidung und einen Gipsverband, sowie die unmagnetischen (austenitischen) Stützmetalle der Osteosynthese. Im elektrischen Kontakt mit diesen wird eine - sekundäre - Spulenanordnung, der so genannte Übertrager, implantiert. Die in dem Übertrager induzierten Elektropotentiale werden so im Bereich der Knochenläsion sowie allgemein in dem an die Osteosynthesemittel angrenzenden Gewebe zur Wirkung gebracht.

Mit dieser Technik der induktiven Übertragung therapeutisch wirksamer Elektropotentiale auf die Bestandteile der Osteosynthese wird die Infektionsgefahr durch percutane Stromleitungen vermieden, und es können die Behandlungsparameter elektrische Spannung, Frequenz, Intensität, Signalform und die Behandlungszeit mit der indikationsspezifischen Programmierung eines Funktionsstrom-Generators des induzierenden Magnetfeldes bestimmt werden.

Ein weiteres Beispiel für ein Implantat, das im Rahmen der Elektro-Osteotherapie eingesetzt werden kann, ist in der DE 23 11 817 C1 und der US 3,918,440 angegeben. Die hier beschriebene Druckknopfschraube wird mit einer schraubenextern angeordneten Übertragerspule über einen Druckknopf kontaktiert und so zum Bestandteil der Elektro-Osteotherapie.

Der Erfindung liegt die Aufgabe zugrunde, eine Möglichkeit zur variantenreichen Einbindung von Implantaten in die Elektro-Osteotherapie zu schaffen, wobei insbesondere eine kompakte Anordnung angestrebt wird, die über die angestrebte Implantationsdauer ohne weiteres haltbar ist.

Diese Aufgabe wird mit den Merkmalen des unabhängigen Anspruches gelöst.

Vorteilhafte Ausführungsformen der Erfindung sind in den abhängigen Ansprüchen angegeben.

Die Erfindung besteht in einer Vorrichtung zum Polarisieren von mindestens zwei räumlich getrennten, elektrisch zumindest teilweise leitfähigen Implantaten nach Anspruch 1.

Im Gegensatz zum bekannten Stand der Technik dienen die Implantate, also nicht nur zu Fixationszwecken und als Elektrode, sondern das erste Implantat dient auch gleichzeitig als Träger der Übertragerspule. Die Gegenelektrode wird von einem anderen baugleichen oder auch völlig andersartigen Implantat dadurch gebildet, dass eine Leitungsverbindung von der von dem ersten Implantat getragenen Spule zu dem anderen Implantat erfolgt, wobei ohne weiteres auch mehrere Implantate durch derartige Leitungsverbindungen kontaktiert werden können, so dass jedes dieser Implantate eine Gegenelektrode zu dem die Spule tragenden Implantat bildet. Alle beteiligten Implantate können baugleich oder verschiedenartig aufgebaut sein.

Die Spule wird dabei in einem Hohlraum des ersten Implantates angeordnet. Die erste Kontakteinrichtung kontaktiert das erste Implantat innerhalb des Hohlraums elektrisch. Damit ist die Spule sicher im menschlichen Körper untergebracht. Insbesondere ist die Verbindung zwischen der Spule und dem ersten Implantat vollkommen sicher und störunanfällig, da diese Verbindung in keiner Weise durch irgendwelche Vorgänge im menschlichen Körper beeinflusst wird.

Es ist nützlich, dass die Spule auf einen magnetisch leitenden Kern gewickelt ist. Auf diese Weise wird die Umsetzung der durch ein externes Magnetfeld aufgebrachten Energie in eine elektrische Spannung effizienter.

Die Erfindung entfaltet ihre Vorteile besonders im Zusammenhang damit, dass das mindestens eine zweite Implantat ebenfalls eine Knochenschraube ist. Knochenschrauben können die unterschiedlichsten Funktionen erfüllen und sind vergleichsweise leicht an den verschiedensten Positionen des Körpers implantierbar. Insofern kann die vorliegende Erfindung im Zusammenhang mit Knochenschrauben besonders flexibel eingesetzt werden.

Die Erfindung ist hier nützlicherweise dadurch weitergebildet, dass die mindestens eine zweite Kontakteinrichtung einen Kontaktdruckknopf aufweist, über den eine mechanische und elektrische Verbindung zu dem mindestens einen zweiten Implantat herstellbar ist. Da es während der Operation um eine einfache Handhabung der zu implantierenden Komponenten geht, stellt die Verbindung der zweiten Kontakteinrichtung mit dem zweiten Implantat über einen Kontaktdruckknopf eine besonders nützliche Lösung dar. Ein Druckknopf ist in der Lage, gleichzeitig eine mechanische sowie eine elektrische Verbindung herzustellen.

Es ist besonders nützlich, dass mindestens ein Isoliermantel vorgesehen ist, durch den der Kontaktdruckknopf der mindestens einen zweiten Kontakteinrichtung gegen umgebendes Gewebe elektrisch isolierbar ist. Hierdurch wird vermieden, dass Knochenwachstum im Bereich des Kontaktdruckknopfes stattfindet, was insbesondere im Hinblick auf die Explantation des Implantates hinderlich sein beziehungsweise zusätzliche aufwendige Maßnahmen erforderlich machen könnte.

Im Sinne einer guten Handhabbarkeit der Komponenten ist wiederrum nützlicherweise vorgesehen, dass die Spule von einem Spulenträger gehalten wird, über den ein mechanischer Kontakt zu dem ersten Implantat herstellbar ist.

Auch dann kann in vorteilhafter Weise dafür Sorge getragen werden, dass mindestens ein Isoliermantel vorgesehen ist, durch den ein proximaler Bereich des Spulenträgers gegen umgebendes Gewebe elektrisch isolierbar ist.

Der Spulenträger ist besonders einfach handhabbar, wenn vorgesehen ist, dass der mechanische Kontakt eine Rastverbindung umfasst.

Es ist von besonderem Vorteil, dass der Spulenträger einen Verteiler aufnimmt, mit dem mehrere zweite Kontakteinrichtungen verbunden sind. Die Leitungen, die zu den verschiedenen zweiten Kontakteinrichtungen führen sind somit sicher innerhalb des Spulenträgers kontaktiert.

Die vorliegende Erfindung kann so weitergebildet sein, dass die Spule Bestandteil einer elektrischen Schaltung ist, die neben der Spule mindestens eine weitere elektrisch wirksame Komponente aufweist. Hierdurch können zusätzliche Funktionalitäten bereitgestellt werden.

Beispielsweise kann vorgesehen sein, dass als weitere elektrisch wirksame Komponente ein Akkumulator vorgesehen ist, der parallel zu der Spule geschaltet ist. Hierdurch ist es möglich, die elektrischen Potentiale der Implantate auch dann aufrecht zu erhalten, wenn gerade kein externes Magnetfeld vorhanden ist. Der Akkumulator wird während der Behandlung mit externem Magnetfeld aufgeladen, wobei hierzu eine Gleichrichtung der induzierten Wechselspannung erforderlich ist. Bei Abwesenheit des externen Magnetfeldes kann der Akkumulator dann die gespeicherte Energie wieder abgeben, indem eine elektrische Spannung und ein damit verbundener Stromfluss erzeugt wird.

Nützlicherweise kann auch vorgesehen sein, dass als weitere elektrisch wirksame Komponente ein Funktionsgenerator vorgesehen ist. Die Signalformung zur Erzeugung der elektrischen Felder im Bereich der Implantate muss also nicht alleine oder auch gar nicht von dem externen Magnetfeld vorgenommen werden. Vielmehr ist es möglich, einen Funktionsgenerator vorzusehen, der diese Aufgabe übernimmt. Besonders nützlich ist dies im Zusammenhang mit einem ebenfalls vorgesehenen Akkumulator, der durch die Spule bei anwesendem externem Magnetfeld aufgeladen wird. Dieser kann die für den Betrieb des Funktionsgenerators erforderliche Spannung dann auch bei abwesendem externen Magnetfeld zur Verfügung stellen.

Es kann ebenfalls nützlich sein, dass als weitere elektrisch wirksame Komponente eine Schaltung vorgesehen ist, die geeignet ist, eine durch die Vorrichtung zur Verfügung gestellte Wechselspannung so zu modifizieren, dass das erste Implantat zumindest überwiegend eine erste Polarität hat, während das zweite Implantat zumindest überwiegend eine zweite Polarität hat, die der ersten Polarität entgegengesetzt ist. Bekanntermaßen hängt die Osteogenese von der Polarität der jeweiligen Elektrode ab. Die Kathode begünstigt die Osteogenese, und die Anode behindert dies. Folglich kann durch geeignete Gleichrichtung je nach Situation das richtige bewirkt werden. Die Schaltung kann diskret oder als integrierte Schaltung, vorzugsweise als ASIC, realisiert sein.

Gemäß einer bevorzugten Ausführungsform der Erfindung ist vorgesehen, dass die weitere elektrisch wirksame Komponente in dem Hohlraum des ersten Implantates angeordnet werden kann. Alle elektrisch wirksamen Komponenten sind somit an einem Ort angeordnet, so dass man sich zur Kontaktierung der zweiten Implantate mit einem bereits beschriebenen Druckknopf begnügen kann.

Es ist aber auch möglich, dass die weitere elektrisch wirksame Komponente in einem Hohlraum des zweiten Implantates angeordnet werden kann. Diese Lösung ist zu bevorzugen, wenn nicht ausreichend Raum im ersten Implantat zur Verfügung steht, um alle Komponenten unterzubringen.

Die Erfindung betrifft weiterhin ein Implantationssystem mit einer erfindungsgemäßen Vorrichtung und mindestens einem zweiten, vom ersten Implantat räumlich getrennt zu implantierenden elektrisch zumindest teilweise leitfähigen Implantat.

Somit wird insbesondere eine elektromagnetisch induzierbare Aufnehmerspule über einem magnetisch leitenden Weicheisenkern vorgeschlagen, die in das Lumen einer im Bereich eines Knochendefektes verankerten Hohlschraube eingeschoben wird. Die Aufnehmerspule ist in eine Isolierschicht aus Epoxydharz oder Silikon gehüllt und wird mit ihrem unteren Ende mit einer Spiral- oder Blechfeder elektrisch leitend verbunden. Mit dieser kontaktiert sie die Knochenschraube beim Einschieben in das Lumen der Schraube. Das obere Ende des Spulenkernes überragt die Spulenwicklung und wird von einer Hülse aus Edelstahl oder Titan aufgenommen und durch Verkleben mit dieser mechanisch festgehalten. Die Hülse besitzt an ihrem oberen Ende die Form eines Druckknopfes, der nach dem Einschieben der Aufnehmerspule in einen Sitz im Kopf der Knochenschraube eingedrückt wird. Das obere Wicklungsende der Aufnehmerspule führt innerhalb der Hülse, von dieser isoliert, zu einer elektrischen Verteilung auf mehrere, beispielsweise 1 bis 3 oder 4 Kabelverbindungen von beispielsweise 3 bis 5 cm Länge, die durch elektrische Druckkontakte jeweils im Kopf weiterer Knochenschrauben, diese gegen die die Aufnehmerspule tragende Schraube polarisiert. Anders als bei der Vorrichtung des "biloparen Induktionsschraubensystems" (siehe unten), bei dem beide Ausgänge der Aufnehmerspule im Lumen einer Knochen-Hohlschraube jeweils mit dem Schaft und einer von diesem isolierten Spitze kontaktiert sind, können für das erfindungsgemäße System der mono- oder unipolaren Induktionsschrauben beliebige Hohlschrauben dienen, wie sie zur Fixierung und Ruhigstellung von Knochenfragmenten Verwendung finden.

Die Hohlschrauben bestehen vorzugsweise aus Titan-Legierungen, an deren Oberfläche sich im Gewebe eine schlecht leitende Oxid-Schicht bildet. Das kann durch die Beschichtung mit hochleitfähigem Titan-Niob-Oxinitrid (Ti, Nb)ON verhindert werden.

Ein weiteres Merkmal bietet die Möglichkeit der Miniaturisierung der Aufnehmerspule. Durch eine Änderung der Steilheit der bei gleicher Frequenz aufsteigenden oder abfallenden Flanken der Sinusschwingungen des induzierenden Elektromagnetfeldes, kann bei gleichbleibender Amplitude die induzierte elektrische Spannung erhöht werden und damit ein Verlust, der durch die Miniaturisierung der Aufnehmerspule entsteht, ausgeglichen werden.

Vom dem bipolaren Induktionsschraubensystem (BISS) nach DE 199 28 449 C1 unterscheidet sich das erfindungsgemäße monopolare Induktionsschraubensystem (MISS) insbesondere durch die räumliche Verteilung des stimulierenden elektrischen Feldes im Knochen. Während der Verlauf des elektrischen Feldes bei der bioplaren Induktionsschraube nur die der Oberfläche der Schraube unmittelbar anliegende Gewebeschicht erreicht, erstreckt sich bei dem erfindungsgemäßen System der monopolaren Induktionsschrauben das induzierte elektrische Feld zwischen diesen über das gesamte Volumen beispielsweise einer ausgedehnten Frakturzone oder eines pathologisch veränderten Knochenareals, das mit Eigen- und Fremdknochen oder Knochenersatzmaterial aufgefüllt und biologisch integriert werden muss. Diesen Anforderungen wird das monopolare Induktionsschraubensystem in idealer Weise gerecht.

Die erfindungsgemäße Vorrichtung ist auch der "Druckknopfschraube" nach DE 23 11 817 C2 und US 3,918,440 deutlich überlegen. Der Nachteil dieser Anordnung der im Weichgewebe implantierten Aufnehmerspulen (der sogenannten Übertrager) mit 3 bis 5 mm Durchmesser und 50 mm Länge, zeigt sich durch eine begrenzte Haltbarkeit ihrer relativ langen Kabelverbindungen von 5-10 cm zu den Osteosyntheseschrauben. Aufgrund ihrer begrenzten Dauerschwingfestigkeit entstanden Kabelbrüche innerhalb der notwendigen Therapiedauer von 3 bis 6 Monaten häufig dann, wenn die Aufnehmehrspulen in das Weichgewebe einwuchsen und dem Spiel der Muskel und Sehnen folgten.

Materialien für die Isoliermasse im Spulenhalter sind zum Beipiel Polyetheretherketon (PEEK) und Polyvinylidenflourid (PVDF). Beide Materialien sind biokompatibel, autoklavierbar und gammastrahlensterilisierbar.

Die Kabelverbindungen bestehen vorzugsweise aus 4-fach gewendeltem Herzschrittmacherdraht MP35N mit Cobalt-Basis-Legierung.

Die Knochenschrauben sind vorzugsweise aus einer Titan-Legierung mit leitwerterhöhender Beschichtung aus Titan-Niob-Oxinitrid (Ti, Nb)ON gefertigt.

Bei den Schrauben des Bipolaren-Induktions-Schrauben-Systems (BISS) bildet sich erfahrungsgemäß im Laufe der therapeutisch notwendigen Behandlungsdauer von 3 bis 5 Monaten und täglichen Induktionszeiten von 2 bis 3 mal 45 Minuten neuer Knochen nicht nur wie beabsichtigt, um den Schaft der Bipolar-Schrauben im Bereich des Knochendefektes, sondern auch in der unmittelbaren Umgebung des Schraubenkopfes. Für die Entnahme der bipolaren Schrauben zusammen mit den anderen Teilen einer Metall-Osteosynthese (Stützplatte, Schrauben, Drähte,...) die orthopädisch regelmäßig ein Jahr nach Implantation erfolgen soll, bildet die sehr harte Knochenschicht um den Kopf der Bipolarschraube eine erhebliche Komplikation. Härte und Dicke dieses Knochens ist nur mit "schwerem" Werkzeug, z.B. Hammer und Meißel zu entfernen. Die Gefahr von Gewebe-Metallosen u.a. durch Metallspäne, die bei dieser Prozedur entstehen, ist oft nicht zu vermeiden.

Die unerwünschte, unter Umständen gefährliche Knochenhülle um den Kontaktbereich (Druckknopf im Schraubenkopf) kann durch einen isolierenden Mantel um den Druckkontaktknopf vermieden werden, dessen Länge nach unten auch den Kopf der kontaktierten Knochenschraube mit umhüllt, in der Weise, dass jeder unmittelbare elektrische Kontakt mit dem Knochen sicher vermieden wird.

Der Mantel kann zweckmäßigerweise Teil der Isolierung des Verteilers im Kopf des Druckknopf-Kontaktes aus PEEK oder PVDF sein und mit seinem oberen Ende beispielsweise in Form einer Pilzkappe den die Aufnehmerspule und den Kabelverteiler enthaltenden Druckknopf -Einsatz verschließen.

Durch die Verschlusskappe führen die Kabelverbindungen zwischen dem Verteiler und den Kontakt- Druckknöpfen zu den weiteren monopolaren Knochenschrauben des Systems, die in gleicher Weise isolierend ummantelt sind.

Die Erfindung wird nun mit Bezug auf die begleitenden Zeichnungen anhand besonders bevorzugter Ausführungsformen beispielhaft erläutert.
- Fig. 1: zeigt eine schematische perspektivische Darstellung eines erfindungsgemäßen Implantationssystems mit zugehörigen Knochenschrauben;
- Fig. 2: zeigt eine schematische perspektivische, teilweise geschnittene Darstellung einer erfindungsgemäßen Polarisationsvorrichtung;
- Fig. 3: zeigt eine schematische perspektivische Darstellung eines erfindungsgemäßen Implantationssystems mit zugehörigen Knochenschrauben;
- Fig. 4: zeigt eine schematische perspektivische, teilweise geschnittene Darstellung einer erfindungsgemäßen Polarisationsvorrichtung;
- Fig. 5: zeigt ein Anwendungsbeispiel eines erfindungsgemäße Implantationssystems;
- Fig. 6: zeigt ein Beispiel für die Anordnung elektrischer Komponenten bei einer erfindungsgemäßen Polarisationsvorrichtung und
- Fig. 7: ein weiteres Beispiel für die Anordnung elektrischer Komponenten bei einer erfindungsgemäßen Polarisationsvorrichtung.

Bei der nachfolgenden Beschreibung der Zeichnungen bezeichnen gleiche Bezugszeichen gleiche oder verlgeichbare Komponenten.

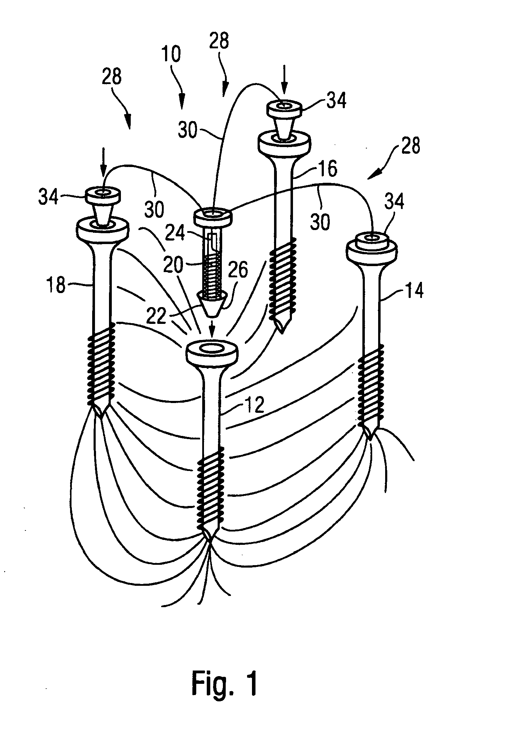

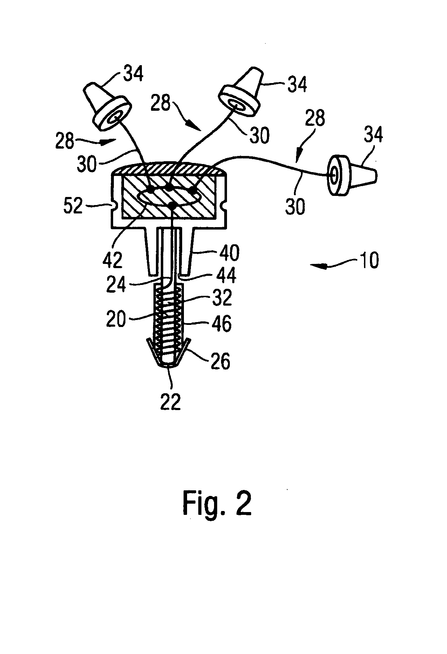

Fig. 1 zeigt eine schematische perspektivische Darstellung eines erfindungsgemäßen Implantationssystems mit zugehörigen Knochenschrauben. Fig. 2 zeigt eine schematische perspektivische, teilweise geschnittene Darstellung einer erfindungsgemäßen Polarisationsvorrichtung. Es sind vier Knochenschrauben 12, 14, 16, 18 sowie eine mit den Knochenschrauben 12, 14, 16, 18 in Verbindung stehende bzw. in Verbindungen tretende Polarisationsvorrichtung 10 dargestellt. Die Polarisationsvorrichtung 10 enthält eine Spule 20, die auf einen magnetisch leitenden Kern 32 gewickelt ist. Ein erster Pol 22 der Spule 20 steht mit einer Kontakteinrichtung 26 in Verbindung, die als Kontaktfeder ausgebildet ist. Ein zweiter Pol 24 der Spule 20 führt zu einem Verteiler 42. An diesem Verteiler 42 sind drei weitere Kontakteinrichtungen 28 angeschlossen. Diese Kontakteinrichtungen 28 umfassen eine elektrische Leitung 30 und Kontaktdruckknöpfe 34 an den dem Verteile 42 abgewandten Enden der Leitungen 30. In Fig. 1 ist die Knochenschraube 14 bereits mit dem zugehörigen Kontaktdruckknopf 34 verbunden, während bezogen auf die Knochenschrauben 16, 18 die zugehörigen Kontaktdruckknöpfe 34 unmittelbar vor ihrer Verbindung mit den Knochenschrauben 16, 18 dargestellt sind. Die Spule 20 wird von einem Spulenträger 40 gehalten, beispielsweise mittelbar dadurch, dass der magnetisch leitende Kern 32, auf den die Spule 20 aufgewickelt ist, in einer Ausnehmung 44 des Spulenträgers kraft- und/oder formschlüssig sitzt. Die Spule ist von einem Isoliermantel 46 umgeben und so dagegen geschützt, anders als über die mit dem ersten Pool 22 verbundene Kontakteinrichtung 26 mit der Knochenschraube in elektrischen Kontakt zu treten. Der Verteiler 42 ist in einer Isoliermasse 48 eingebettet, beispielsweise bestehend aus PEEK oder PVDF. Das Gehäuse 50, das die Ausnehmung 44 zum Halten der Spule 20 bzw. des magnetisch leitenden Kerns 22 aufweist, hat eine zumindest abschnittsweise umlaufende Nut 52. Die Knochenschraube 12, die mit der ersten Kontakteinrichtung 26 kontaktiert werden soll, weist eine entsprechende Komplementärvorrichtung aus, also dass eine stabile Rastverbindung zwischen dem Gehäuse 50 und der Knochenschraube 12 zur Verfügung gestellt werden kann.

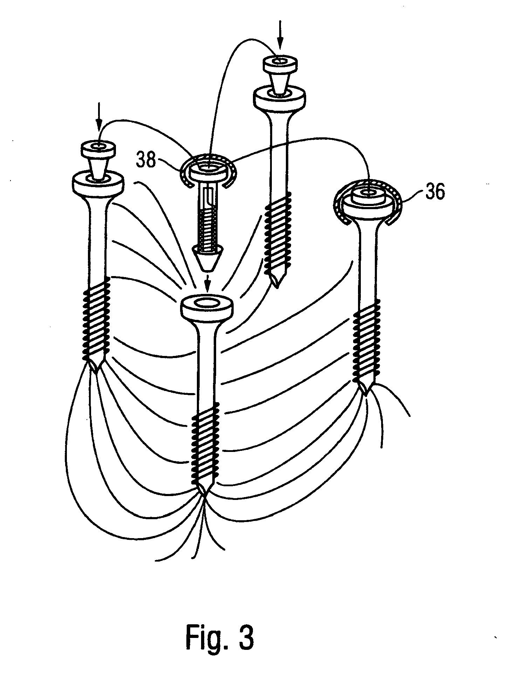

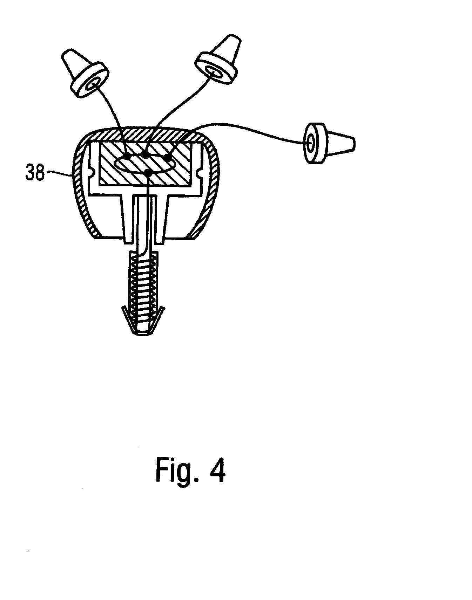

Fig. 3 zeigt eine schematische perspektivische Darstellung eines erfindungsgemäßen Implantationssystems mit zugehörigen Knochenschrauben. Fig. 4 zeigt eine schematische perspektivische, teilweise geschnittene Darstellung einer erfindungsgemäßen Polarisationsvorrichtung. Hier ist zusätzlich dargestellt, dass die Bereiche der Polarisationsvorrichtung, über die die Kontakteinrichtungen mit den Knochenschrauben gekoppelt werden, von Isoliermänteln 36, 38 umgeben sein können. Diese Isoliermäntel 36, 38 verhindern einen stimulierten Knochenaufbau um die isolierten Bereiche, so dass eine Explantation der Polarisationsvorrichtung erleichtert wird.

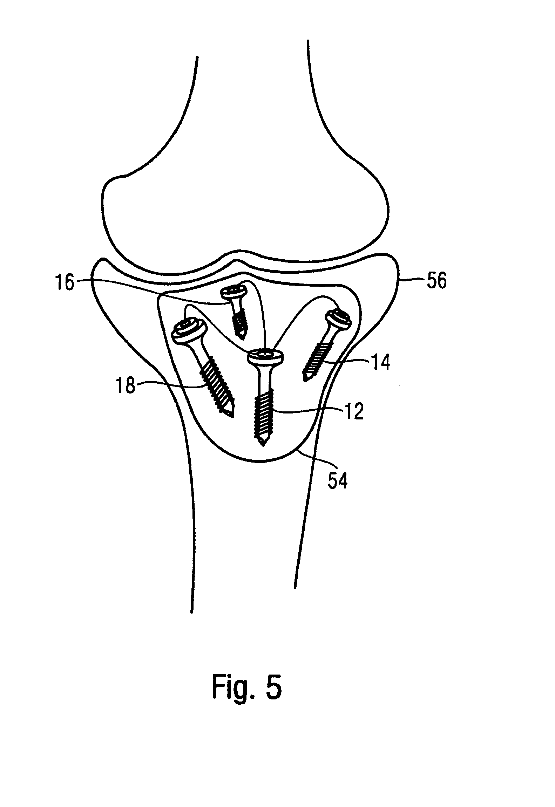

Fig. 5 zeigt ein Anwendungsbeispiel eines erfindungsgemäßen Implantationssystems. Es ist dargestellt, wie eine erfindungsgemäße Polarisationsvorrichtung im Bereich eines Tumors 54 im Tibiakopf 56 angeordnet sein kann.

Ein weiteres nicht bildlich dargestelltes Anwendungsbeispiel ist die Schraubenosteosynthese medialer Schenkelhalsfrakturen, bei der mehrere, beispielsweise drei Schrauben zum Einsatz kommen, die in Gestalt der erfindungsgemäßen Polarisationsvorrichtung funktional mit einander verbunden sein können.

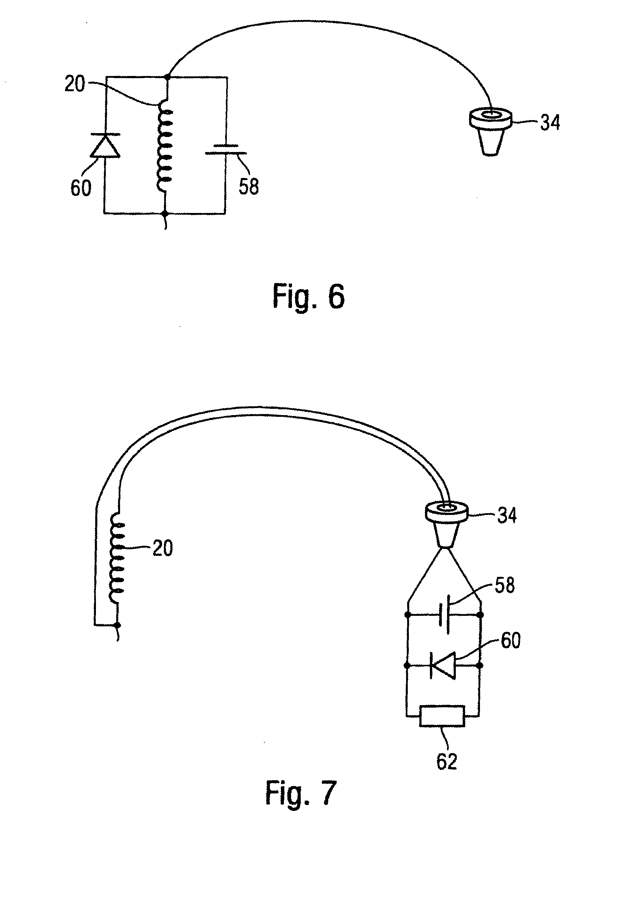

Fig. 6 zeigt ein Beispiel für die Anordnung elektrischer Komponenten bei einer erfindungsgemäßen Polarisationsvorrichtung. Hier ist dargestellt, dass parallel zur Spule 20 ein Akkumulator 58 angeordnet sein kann. Um dem Akkumulator 58 zu dessen Aufladung eine Gleichspannung zur Verfügung zu stellen, ist ferner ein Gleichrichter 60, der hier durch ein Diodensymbol dargestellt ist, zu der Anordnung aus Akkumulator 58 und Spule 20 parallel geschaltet. Der Akkumulator 28 ist in räumlicher Nähe zur Spule 20 platziert, so dass dieser, wenn ausreichend Raum zur Verfügung steht, wie die Spule 20 im Hohlraum eines Implantats angeordnet wird. Zu den Kontaktdruckknöpfen 34, von denen einer beispielhaft dargestellt ist, muss dann nur eine einzige elektrische Verbindung führen.

Fig. 7 zeigt ein weiteres Beispiel für die Anordnung elektrischer Komponenten bei einer erfindungsgemäßen Polarisationsvorrichtung. Hier ist der Akkumulator 58 zwar auch elektrisch parallel zur Spule 20 angeordnet, allerdings in der Nähe des Kontaktdruckknopfes 34. Diese Lösung ist bevorzugt, wenn der Hohlraum des Implantats, das die Spule 20 aufnimmt, für den Akkumulator 58 und die Spule 20 nicht ausreicht. Dann wird der Akkumulator 58 vorzugsweise in einem anderen Implantat untergebracht. In diesem Fall müssen zwei elektrische Leitungen aus dem Bereich des Implantats, das die Spule 20 trägt, zu dem Implantat, das den Akkumulator 58 trägt, geführt werden, wobei dies selbstverständlich durch ein einziges Kabel bewerkstelligt werden kann. Die Parallelschaltung eines Akkumulators 58 zur Spule 20 ermöglicht es, den Akkumulator 58 dann zu laden, wenn sich die Spule 20 in einem externen Magnetfeld befindet. Auch in Abwesenheit des externen Magnetfeldes kann dann durch den Akkumulator 58 eine elektrische Spannung zwischen den Implantaten aufrecht erhalten werden. Allerdings kann ohne weitere Maßnahmen ohne externes Magnetfeld nur eine Gleichspannung durch den Akkumulator 58 zur Verfügung gestellt werden. Dient der Akkumulator 58 allerdings als Spannungslieferant für einen Funktionsgenerator 62 wie es in Fig. 7 dargestellt ist, so kann dieser durch geeignete Kontaktierungen seiner Ausgänge mit den elektrisch leitfähigen Bereichen der Implantate (hier nicht dargestellt) eine Wechselspannung auch in Abwesenheit eines externes Magnetfeldes liefern.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste

- 10: Polarisationsvorrichtung
- 12: Implantat, Knochenschraube
- 14: Implantat, Knochenschraube
- 16: Implantat, Knochenschraube
- 18: Implantat, Knochenschraube
- 20: Spule
- 22: Pol
- 24: Pol
- 26: Kontakteinrichtung
- 28: Kontakteinrichtung
- 30: Leitung
- 32: magnetisch leitender Kern
- 34: Kontaktdruckknopf
- 36: Isoliermantel
- 38: Isoliermantel
- 40: Spulenträger
- 42: Verteiler
- 44: Ausnehmung
- 46: Isoliermantel
- 48: Isoliermasse
- 50: Gehäuse
- 52: Nut
- 54: Tumor
- 56: Tibiakopf
- 58: Akkumulator
- 60: Gleichrichter
- 62: Funktionsgenerator

## Patentansprüche

1. Vorrichtung (10) zum Polarisieren von mindestens zwei räumlich getrennten, elektrisch zumindest teilweise leitfähigen Implantaten (12, 14, 16, 18), mit
- einem ersten Implantat (12),
- einer Spule (20), die einen ersten Pol (22) und einen zweiten Pol (24) aufweist,
- einer dem ersten Pol (22) zugeordneten ersten Kontakteinrichtung (26) zum elektrischen Kontaktieren des ersten Implantates (12),
- mindestens einer dem zweiten Pol (24) zugeordneten zweiten Kontakteinrichtung (28) zum elektrischen Kontaktieren mindestens eines zweiten Implantates (14, 16, 18),
- wobei die mindestens eine zweite Kontakteinrichtung (28) eine flexible elektrische Leitung (30) umfasst, die eine Kontaktierung des mindestens einen zweiten Implantates (14, 16, 18) ermöglicht, so dass die Spule (20) räumlich getrennt von dem mindestens einen zweiten Implantat (14, 16, 18) angeordnet werden kann,
**dadurch gekennzeichnet, dass**
- die Spule (20) unmittelbar in dem ersten Implantat (12) angeordnet und von diesem getragen werden kann,
- wobei die Spule (20) in einem Hohlraum des ersten Implantates (12) angeordnet werden kann und die erste Kontakteinrichtung (26) das erste Implantat (12) innerhalb des Hohlraums elektrisch kontaktiert und
- wobei das erste Implantat (12) eine Knochenschraube ist.

2. Vorrichtung (10) nach Anspruch 1, **dadurch gekennzeichnet, dass** die Spule (20) auf einen magnetisch leitenden Kern (32) gewickelt ist.

3. Vorrichtung (10) nach Anspruch 1 oder 2, **dadurch gekennzeichnet, dass** das mindestens eine zweite Implantat (14, 16, 18) eine Knochenschraube ist.

4. Vorrichtung (10) nach einem der vorhergehenden Ansprüche, **dadurch gekennzeichnet, dass** die mindestens eine zweite Kontakteinrichtung (28) einen Kontaktdruckknopf (34) aufweist, über den eine mechanische und elektrische Verbindung zu dem mindestens einen zweiten Implantat (14, 16, 18) herstellbar ist.

5. Vorrichtung (10) nach Anspruch 4, **dadurch gekennzeichnet, dass** mindestens ein Isoliermantel (36) vorgesehen ist, durch den der Kontaktdruckknopf (34) der mindestens einen zweiten Kontakteinrichtung (28) gegen umgebendes Gewebe elektrisch isolierbar ist.

6. Vorrichtung (10) nach einem der vorhergehenden Ansprüche, **dadurch gekennzeichnet, dass** die Spule (20) von einem Spulenträger (40) gehalten wird, über den ein mechanischer Kontakt zu dem ersten Implantat (12) herstellbar ist.

7. Vorrichtung (10) nach Anspruch 6, **dadurch gekennzeichnet, dass** mindestens ein Isoliermantel (38) vorgesehen ist, durch den ein proximaler Bereich des Spulenträgers (40) gegen umgebendes Gewebe elektrisch isolierbar ist.

8. Vorrichtung (10) nach Anspruch 6 oder 7, **dadurch gekennzeichnet dass** der mechanische Kontakt eine Rastverbindung umfasst.

9. Vorrichtung (10) nach einem der Ansprüche 6 bis 8, **dadurch gekennzeichnet, dass** der Spulenträger (40) einen Verteiler (42) aufnimmt, mit dem mehrere zweite Kontakteinrichtungen (28) verbunden sind.

10. Vorrichtung (10) nach einem der vorhergehenden Ansprüche, **dadurch gekennzeichnet, dass** die Spule Bestandteil einer elektrischen Schaltung ist, die neben der Spule mindestens eine weitere elektrisch wirksame Komponente aufweist.

11. Vorrichtung (10) nach Anspruch 10, **dadurch gekennzeichnet, dass** als weitere elektrisch wirksame Komponente ein Akkumulator vorgesehen ist, der parallel zu der Spule geschaltet ist.

12. Vorrichtung (10) nach Anspruch 10 oder 11, **dadurch gekennzeichnet, dass** als weitere elektrisch wirksame Komponente ein Funktionsgenerator vorgesehen ist.

13. Vorrichtung (10) nach Ansprüchen 10 oder 12, **dadurch gekennzeichnet, dass** als weitere elektrisch wirksame Komponente eine Schaltung vorgesehen ist, die geeignet ist, eine durch die Vorrichtung zur Verfügung gestellte Wechselspannung so zu modifizieren, dass das erste Implantat zumindest überwiegend eine erste Polarität hat, während das zweite Implantat zumindest überwiegend eine zweite Polarität hat, die der ersten Polarität entgegengesetzt ist.

14. Vorrichtung (10) nach einem der Ansprüche 10 bis 13, **dadurch gekennzeichnet, dass** die weitere elektrisch wirksame Komponente in dem Hohlraum des ersten Implantates (12) angeordnet werden kann.

15. Vorrichtung (10) nach einem der Ansprüche 10 bis 14, **dadurch gekennzeichnet, dass** die weitere elektrisch wirksame Komponente in einem Hohlraum des zweiten Implantates (14, 16, 18) angeordnet werden kann.

16. Implantationssystem mit einer Vorrichtung (10) nach einem der Ansprüche 1 bis 15 und mindestens einem zweiten, vom ersten Implantat räumlich getrennt zu implantierenden, elektrisch zumindest teilweise leitfähigen Implantat (14, 16, 18).

## Claims

1. An apparatus (10) for polarizing at least two spatially separate, electrically at least partially conductive implants (12, 14, 16, 18), comprising
- a first implant (12),
- a coil (20) having a first pole (22) and a second pole (24),
- a first contact device (26) associated with the first pole (22), for electrically contacting the first implant (12),
- at least one second contact device (28) associated with the second pole (24), for electrically contacting at least a second implant (14, 16, 18),
- wherein the at least one second contact device (28) comprises a flexible electrical conductor (30) which enables contacting the at least one second implant (14, 16, 18) so that the coil (20) can be placed at a distance from the at least one second implant (14, 16, 18),
**characterized in that**
- the coil (20) is arranged directly in the first implant (12) and can be supported by it,
- wherein the coil (20) can be placed inside a cavity of the first implant (12) and the first contact device (26) electrically contacts the first implant (12) inside the cavity and
- wherein the first implant (12) is a bone screw.

2. The apparatus (10) of claim 1, wherein the coil (20) is wound on a magnetically conductive core (32).

3. The apparatus (10) of claim 1 or 2, wherein the at least one second implant (14, 16, 18) is a bone screw.

4. The apparatus (10) of one of the preceding claims, wherein the at least one second contact device (28) comprises a contact press button (34) for enabling establishing a mechanical and electrical link to the at least one second implant (14, 16, 18).

5. The apparatus (10) of claim 4, wherein there is at least one isolating jacket (36) by means of which the contact press button (34) of the at least one second contact device (28) can be electrically isolated from a surrounding tissue.

6. The apparatus (10) of one of the preceding claims, wherein the coil (20) is supported by a coil carrier (40) by means of which a mechanical contact to the first implant (12) can be established.

7. The apparatus (10) of claim 6, wherein there is at least one isolating shell (38) by means of which a proximal region of the coil carrier (40) can be electrically isolated from a surrounding tissue.

8. The apparatus (10) of claim 6 or 7, wherein the mechanical contact comprises a click-stop link.

9. The apparatus (10) of one of claims 6 to 8, wherein the coil carrier (40) accommodates a distributer (42) to which several second contact devices (28) are connected.

10. The apparatus (10) of one of the preceding claims, **characterized in that** the coil is part of an electrical circuit which comprises at least one further electrically effective component in addition to the coil.

11. The apparatus (10) of claim 10, wherein an accumulator connected parallel to the coil is provided as the further electrically effective component.

12. The apparatus (10) of claim 10 or 11, wherein a function generator is provided as the further electrically effective component.

13. The apparatus (10) of claim 10 or 12, **characterized in that** the further electrically effective component is a circuit for modifying an alternating voltage provided by the apparatus in such a way that the first implant has at least mainly a first polarity whereas the second implant has at least mainly a second polarity opposed to the first polarity.

14. The apparatus (10) of one of claims 10 to 13, wherein the further electrically effective component can be placed inside the cavity of the first implant (12).

15. The apparatus (10) of one of claims 10 to 14, wherein the further electrically effective component can be placed inside a cavity of the second implant (14, 16, 18).

16. Implantation system comprising an apparatus (10) of one of claims 1 to 15 and at least one at least partially electrically conductive implant (14, 16, 18) to be implanted at a distance from the first implant.

## Revendications

1. Dispositif (10) pour polariser au moins deux implants (12, 14, 16, 18) espacés, qui sont au moins partiellement conducteur d'électricité, le dispositif comportant
- un premier implant (12),
- une bobine (20), qui comporte un premier pôle (22) et un deuxième pôle (24);
- un premier dispositif de contact (26) associé au premier pôle (22), pour contacter électriquement le premier implant (12),
- au moins un deuxième dispositif de contact (28) associé au deuxième pôle (24) pour contacter électriquement au moins un deuxième implant (14, 16, 18),
- l'au moins un deuxième dispositif de contact (28) comportant un conducteur (30) électrique flexible, qui permet de contacter l'au moins un deuxième implant (14, 16, 18) de façon que la bobine (20) peut être placée à une distance de l'au moins un deuxième implant (14, 16, 18),
**caractérisé en ce que** la bobine (20) peut être placée directement dans le premier implant (12) et peut être portée par celui-ci,
- la bobine (20) peut être agencée dans une cavité du premier implant (12) et le premier dispositif de contact (26) est relié électriquement au premier implant (12) à l'intérieur de la cavité et
- le premier implant (12) est une vis d'os.

2. Dispositif (10) selon la revendication 1, **caractérisé en ce que** la bobine (20) est bobinée sur un noyau (32) conducteur de magnétisme.

3. Dispositif (10) selon la revendication 1 ou 2, **caractérisé en ce que** l'au moins un deuxième implant (14, 16, 18) est une vis d'os.

4. Dispositif (10) selon l'une des revendications précédentes, **caractérisé en ce que** l'au moins un deuxième dispositif de contact (28) comporte un bouton-poussoir de contact (34) qui permet d'établir une liaison mécanique et électrique vers l'au moins un deuxième implant (14, 16, 18).

5. Dispositif (10) selon la revendication 4, **caractérisé en ce qu'**au moins une gaine isolante (36) et prévue, qui permet d'isoler électriquement le bouton-poussoir de contact (34) de l'au moins un deuxième dispositif de contact (28) contre du tissu entourant.

6. Dispositif (10) selon l'une des revendications précédentes, **caractérisé en ce que** la bobine (20) est portée par un porteur de bobine (40) qui permet d'établir un contact mécanique vers le premier implant (12).

7. Dispositif (10) selon la revendication 6, **caractérisé en ce qu'**au moins une gaine isolante (38) est prévue, qui permet d'isoler électriquement une zone proximale du porteur de bobine (40) contre du tissu entourant.

8. Dispositif (10) selon la revendication 6 ou 7, **caractérisé en ce que** le contact mécanique comporte un joint d'encliquetage.

9. Dispositif (10) selon l'une des revendications 6 à 8, **caractérisé en ce que** le porteur de bobine (40) admet un répartiteur (42) qui est relié à plusieurs deuxièmes dispositifs de contact (28).

10. Dispositif (10) selon l'une des revendications précédentes, **caractérisé en ce que** la bobine fait partie d'un circuit électrique qui comporte, en outre de la bobine, au moins un autre composant qui est électriquement effectif.

11. Dispositif (10) selon la revendication 10, **caractérisé en ce que** l'autre composant électriquement effectif est un accumulateur mis en parallèle avec la bobine.

12. Dispositif (10) selon la revendication 10 ou 11, **caractérisé en ce que** l'autre composant électriquement effectif est un générateur de fonction.

13. Dispositif (10) selon la revendication 10 ou 12, **caractérisé en ce que** l'autre composant électriquement effectif est un circuit qui est propre à modifier la tension alternante fournie par le dispositif de sorte que le premier implant présente, au moins la plupart du temps, une premier polarité tandis que le deuxième implant présente, au moins la plupart du temps, une deuxième polarité qui est opposée à la première polarité.

14. Dispositif (10) selon l'une des revendications 10 à 13, **caractérisé en ce que** l'autre composant électriquement effectif peut être agencé dans la cavité du premier implant (12).

15. Dispositif (10) selon l'une des revendications 10 à 14, **caractérisé en ce que** l'autre composant électriquement effectif peut être agencé dans une cavité du deuxième implant (14, 16, 18).

16. Système d'implantation qui comporte un dispositif (10) selon l'une des revendications 1 à 15 et au moins un deuxième implant (14, 16, 18), qui est au moins partiellement conducteur d'électricité et à implanter à un endroit séparé du premier implant.
